# EUROPÄISCHE PATENTANMELDUNG

(11) **EP 1 792 573 A1**
(43) Veröffentlichungstag der Anmeldung: **06.06.2007**
(21) Anmeldenummer: 05026198.1
(22) Anmeldetag: 01.12.2005
(51) Int. Cl.: A61B 10/00

(54) **WC-Anlage zur Auswertung von Exkrementen und Ermittlung von Körperzuständen**

(71) Anmelder: Herbst, Martin, A-5090 Lofer (AT)
(72) Erfinder: Herbst, Martin, A-5090 Lofer (AT)

(57) **Zusammenfassung**

Die Erfindung ist eine WC-Anlage, bei der Körperausscheidungen aufgefangen und über Messsensoren geleitet, verschiedenste Messergebnisse geliefert werden. Die gewonnen Messdaten werden automationsunterstützt verarbeitet und ausgewertet. Aufgrund dieser Daten können Gesundheits/Ernährungs- und Körperwerte geliefert und Ratschläge/Maßnahmen über Gesundheit, Sport und Ernährung erstellt werden. Messwerte sind z.B. der Säure-, Ph-, Zuckerwert u.v.a. Werte. Diese Messwerte werden z.B. an einen PC gesendet und werden dort ausgewertet. Die ermittelten Daten geben dann Rückschlüsse über den augenblicklichen körperlichen Zustand desjenigen, der z.B. hineinuriniert hat. Der PC ermittelt für den idealen körperlichen Zustand, bezogen auf vorzuwählenden Daten wie Alter, Gewicht, medizinischer Zustand des Probanden und aller anderer möglichen Einflüsse. Um in diesen gewünschten Idealzustand zu gelangen, wählt der PC aus einer Datenbank die Medikation, insbesondere die Ernährung, Getränke und Speisen aus und in weiterer Folge auch die Art und Weise der Bewegung, des herbeizuführenden Kalorienverbrauchs und zukünftigen Lebensart (Sport, Bewegung, Schlaf, Belastung usw.). Nach Einnahme der vorgeschlagenen Medikation, Getränke oder Speisen, sowie der vorgeschlagenen Betätigung bzw. Bewegung des Körpers, wird der optimale Wohlfühlzustand und somit die Normalwerte für den jeweiligen Körper erreicht.

## Beschreibung

Die Erfindung bezieht sich auf eine WC-Anlage, bei der Körperausscheidungen (fest, flüssig oder gasförmig) mittels einer Auffangvorrichtung aufgefangen, die Körperausscheidungsprodukte über Messsensoren transportiert bzw. geleitet und ausgewertet werden. Anwendung findet die Erfindung im Gebiet Wellness/Gesundheit/Medizin. Die gewonnen Messdaten werden automationsunterstützt verarbeitet. Die Messsensoren sind imstande über mehrere hunderte Messergebnisse zu liefern. Messwerte sind z.B. der Säure-, Ph- oder Zuckerwert, sowie Zellstoffe in fester, flüssiger und gasförmiger Form. Diese Messwerte werden an einen PC bzw. Rechner gesendet und werden dort ausgewertet. Die ermittelten Daten geben dann Rückschlüsse über den augenblicklichen körperlichen Zustand desjenigen, der hineinuriniert hat. Der PC bzw. Rechner ermittelt für den idealen körperlichen Zustand, bezogen auf vorzuwählenden Daten wie Alter, Gewicht, medizinischer Zustand des Probanden und aller anderer möglichen Einflüsse. Um in diesen gewünschten Idealzustand zu gelangen, wählt der PC aus einer Datenbank die Medikation, insbesondere die Ernährung, Getränke und Speisen aus und in weiterer Folge auch die Art und Weise der Bewegung, des herbeizuführenden Kalorienverbrauchs und zukünftigen Lebensart (Sport, Bewegung, Schlaf, Belastung usw.). Nach Einnahme der vorgeschlagenen Medikation, Getränke oder Speisen, sowie der vorgeschlagenen Betätigung bzw. Bewegung des Körpers, wird der optimale Wohlfühlzustand und somit die Normalwerte für den jeweiligen Körper erreicht. Nachfolgend ergibt eine Berechnung die zukünftige Leistungsfähigkeit in Bezug auf Kraft, Ausdauer in physischer und psychischer Hinsicht und eine Berechnung der Lebenserwartung unter Anführung aller lebensnahen Parameter incl. Umwelteinflüsse und Belastung des Körpers. Sinngemäß gilt dies auch für die Auswertung von Kot. Mit dieser Erfindung können auch kritische Gesundheitszustände angezeigt werden. Es kann z.B. frühzeitig der Beginn oder aber auch das Ende einer Krankheit angezeigt werden.

Die Grundelemente der Erfindung sind einzeln betrachtet, allgemein bekannt. Benötigt werden ein WC bzw. Pissoir, Sensoren, die den Urin, Kot bzw. Körpersausscheidungen auswerten, sowie eine automationsunterstützte EDV und die wissenschaftlichen Informationen aus der Medizin, der Ernährungswissenschaft und der Sport/Bewegungswissenschaft.

Bisher ist es äußerst umständlich, aktuelle Messwerte über den Körperzustand zu bekommen, insbesondere über die Ausscheidungsprodukte des menschlichen Körpers, den Urin und den Kot. Hiezu bietet sich derzeit die Möglichkeiten der Auswertung bei einem Hausarzt, der die Urin- bzw. Kotprobe in ein Labor schicken muss und dann die Messwerte ausgewertet zurückerhält oder es sind Streifen in einer Apotheke erhältlich, die über einen bestimmten Messwert des menschlichen Körpers Auskunft geben können. Sind die Messwerte bekannt, erhält man in der Praxis oft nur unzureichend Information darüber, welche Getränke und Nahrungsmittel eingenommen werden sollen, um damit den gewünschten Erfolg zu erhalten. Hausärzte sind keine Ernährungswissenschafter. Hinzu kommt, um das Ergebnis und die aktuellen Messwerte zu kontrollieren, dass ein erneuter Arztbesuch bzw. ein neuer Streifen nötig ist. Die anderen Grundelemente (WC, Pissoir, automationsunterstützte EDV) mit diesen Messwerten, Daten bzw. Informationen vereint, hat es bis dato in dieser Form nicht gegeben. Insbesondere wird der Effekt der Selbstheilung, im Körper bestens unterstützt, um auch problematische Körper in den ursprünglichen Zustand zurückzuversetzen.

Ziel der Erfindung ist eine Herstellung eines Systems, das den Menschen in die Lage versetzt, schnell und ohne großen Aufwand bei Benützung des WC bzw. Pissoirs Informationen über seinen Körper und zugleich eine Empfehlung über jene Lebensmittel, Medikation und Bewegungsabläufe zu erhalten, die bei Einnahme bzw. Durchführung eine entsprechende positive Wirkung und lebensverlängernde Wirkung ausüben und die Messwerte in den gewünschten optimalen Zustand (Wohlfühlzustand) bringt. Die nachgeschaltete Rechnereinheit nimmt Auswertungen aufgrund ernährungswissenschaftlichermedizinischer- und sportwissenschaftlicher Kenntnissen, sowie aller jeweils verfügbarer Messwerte vor. So können Auswertungen generiert werden, wie z.B. Abweichungen von Normalwerten, aber es kann auch in weiterer Folge ein Menü erstellt werden, um die gemessenen Körperwerte in den Normalzustand, bzw. in den gewünschten Köperzustand (Wohlfühlzustand) hinüberzuführen.

Die Vorteile der Erfindung lassen sich wie folgt zusammenfassen:
- Die Urin- bzw. Kotprobe kann einfach abgegeben werden, durch die Benützung eines Pissoirs bzw. WC. Ein Arztbesuch ist nicht mehr nötig.
   Es muss in der Apotheke kein Messstreifen besorgt werden.
- Um jedoch in weiterer Folge die erhaltenen Daten an Gesundheitsdatenbanken sowie nachgeschaltenen Ärzte oder Berater weiterzuleiten, kann dies über Breitbanddatenleitungen (Internet) vernetzt werden.
- Die Messwerte sind schnell zu erhalten. Der Arzt hingegen benötigt ein Labor, das ihm die Messwerte liefert. Bis das Messergebnis des Arztes vorliegt, vergeht Zeit und es ist unter Umständen ein neuer Arztbesuch notwendig.
- Die Auswertung der Exkremente kann bei dieser Erfindung beliebig oft wiederholt werden. Es ist kein erneuter Arztbesuch mehr nötig.
- Man erhält eine Liste mit empfohlenen Lebensmitteln, Getränke, Medikamente und Bewegungsprogramm, die den Körper in den gewünschten Normbereich bringt.
- Die Person kann anhand der Messwerte und unter Berücksichtung der nahrungswissenschaftlichen Kenntnisse aus einer Liste jene Lebensmittel auswählen.
- Sind die Messwerte im gewünschten Normalbereich, entsteht ein Wohlfühleffekt.
- Anhand der Messwerte können Gesundheitszustände ermittelt werden. So können frühzeitig Krankheiten erkannt werden.
- Die Erfindung kann überall eingesetzt werden, wo Platz für ein Pissoir bzw. WC vorgesehen ist, im speziellen bei Arztpraxen, Gastronomiebetrieben, aber auch für zu Hause.
- Für zukünftige Lebenssituationen von Personen können im Vorhinein schon bestimmte Körperwerte so angepasst werden, dass ein erhöhter Ausstoß von Glücks- Wachstumshormonen eintritt. Die Folge daraus ist, dass die entsprechende Person bei Verhandlungen mehr Geschick und Wissenskraft aufweist, welche durch einfache Sauerstoffflutung des Blutes, einhergehend mit einer kalorienarmen und eiweißreichen Ernährung bei Einrechnung der vorgegebenen Zeit, erreicht wird.
- Beseitigung langfristiger körperlicher Zustände wie Rheuma, Übersäuerung des Körpers, welche einen langfristigen und über Monate hinweg einer bestimmten Ernährung/Medikation bedürfen. Es kann so exakt auf die optimale Verträglichkeit eingegangen werden, da bei jedem Urinieren eine Auswertung und Erfolgsrechung in Sekunden durchgeführt werden kann.

Im folgenden wird die Erfindung anhand eines Ausführungsbeispiels und mit Bezug auf die beigefügten Zeichnungen veranschaulicht:
Fig. 1: Seitenansicht eines WC-Spülbeckens mit erfindungsgemäßem Einbau der Urinmessvorrichtung
Fig. 2: Draufsicht auf ein WC-Spülbecken mit erfindungsgemäßem Einbau der Urinmessvorrichtung

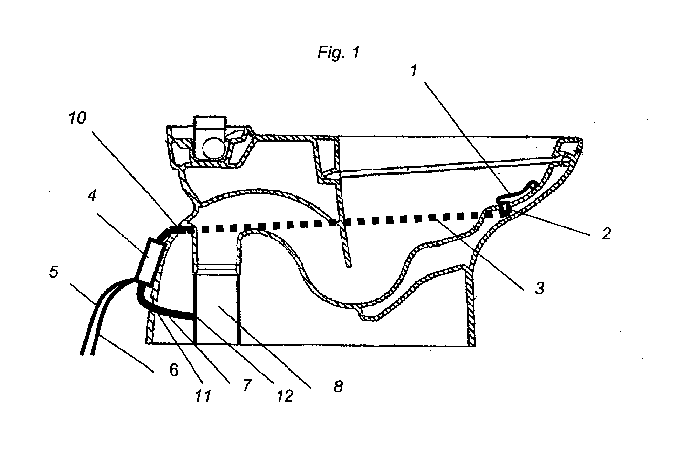

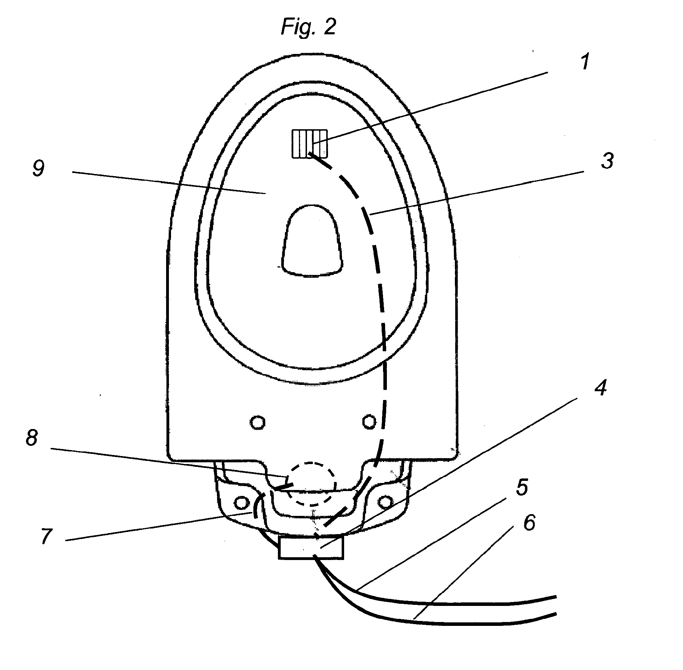

In Fig. 1 und 2 wird ein erfindungsgemäßer Einbausatz verwendet. Dieser umfasst 5 parallel liegende Auffangbehälter 1. Diese Auffangbehälter werden auf der Innenseite der WC-Muschel 9 befestigt, sodass durch diese Behälter Urin aufgefangen wird. Je nach WC-Type können diese Behälter 5 flexibel oder Steif sein, sie können ebenfalls mit oder ohne Sensoren sein. Am unteren Ende der Röhrchen 1 wird durch die WC-Muschel 9 ein Loch 2 gebohrt. Weiters wird ein Loch 10 hinten am WC-Spülbecken gebohrt. Durch diese beiden Löcher führt ein Schlauch 3, der aufgrund eines leichten Gefälles den Urin von Loch 2 zu Loch 3 transportiert. Hinten am WC-Spülbecken befindet sich die Messstation 4 mit den nötigen Urin-Sensoren. Der Urin wird über den Schlauch 3 in Messstation geleitet. Die Sensoren in der Messstation analysieren den Urin auf PH-, Zucker-, Säure- u.v.a. Werte. So lassen sich alle chemischen Zustände nach dem Stand der Technik bereits ermitteln, dies sind derzeit mehr als 400 Messwerte. Von der Messstation wird das Signal z.B. via seriellem Kabel 5 oder Ethernetkabel 6 zu einer nachgeschalteten Rechnereinheit geführt. Die nachgeschaltete Rechnereinheit bzw. der PC übernimmt die Auswertung und Aufbereitung des Messergebnisses. Ist die Messstation 4 mit der Messung fertig, werden die Messrückstände des Urin durch den Schlauch 7 durch die Bohrungen 11 und 12 zurück in den WC-Abfluss 8 geleitet und entsorgt. Sämtliche Bohrungen werden entsprechend abgedichtet. Die nachgeschaltete Rechnereinheit nimmt Auswertungen aufgrund sportwissenschaftlicherer, ernährungswissenschaftlicher und medizinischer Kenntnisse vor. Es können Auswertungen generiert werden, wie z.B. Abweichungen von Normalwerten, aber es kann auch in weiterer Folge ein Menü erstellt werden. Die Bedienung des Systems kann voll- oder halbautomatisch, aber auch manuell erfolgen. Das System kann auch in einem Pissoir eingebaut werden. In weiterer Folge können auch WC-Spülbecken erzeugt, werden, die das Messsystem bereits integriert haben.

Für die gewerbliche Nutzung ergeben sich vielfache Verwendungsmöglichkeiten, überall dort, wo ein WC vorhanden ist, bzw. ein WC angeschlossen werden kann. Anwendung findet es in Arztpraxen sowie in allen Gesundheitseinrichtungen, Hotels, Restaurants, aber auch im privaten Bereich zu Hause.
Bei Restaurants kann aufgrund des Messergebnisses gleich ein Menüvorschlag erstellt werden und auf Wusch sofort zubereitet werden.
In Arztpraxen werden die Messungen einfach und schnell durchgeführt. Die Ärzte erhalten umgehend die Messwerte. Für ganz spezielle Messwerte kann das WC vom Fachpersonal entsprechend vorbereitet und manuell bedient werden.
Von Zuhause aus können z.B. Patienten ihre Körperwerte kontrollieren und sich ernährungsbewusst und gesundheitsoptimal ernähren.

## Patentansprüche

1. WC-Anlage, bei der Körperausscheidungen im flüssigen, festen oder gasförmigen Zustand mittels einer Auffangvorrichtung aufgefangen, die Körperausscheidungen über Messsensoren transportiert bzw. geleitet und ausgewertet werden. Die gewonnen Messdaten werden automationsunterstützt verarbeitet und aufgrund dieser Daten können Gesundheits/Ernährungs/Körperwerte geliefert und Maßnahmen für Gesundheit, Ernährung, Bewegung und Sport erstellt werden. Nach dem Messvorgang werden die Körperausscheidungen entsorgt.

2. WC-Anlage nach Anspruch 1, wobei diese aus einem WC-Spülbecken mit einer Auffangvorrichtung 1 für Urin besteht. Der Urin wird über Schläuche zu einer Messstation geleitet und ausgewertet und nach der Auswertung zurück in den WC-Abfluss 8 geleitet. Die Messstation 4 ist mit Datenleitungen 5 u. 6 mit einem PC verbunden, der über eine Software die Messergebnisse auswertet und Empfehlungen für die Herstellung des gewünschten Körperzustandes erstellt.

3. Eine WC-Anlage nach Anspruch 1 bis 2, wobei diese aus einem Pissoir besteht.

4. Eine WC-Anlage nach Anspruch 1 bis 3, wobei für die automationsunterstützte Datenverarbeitung ein Rechner oder ein Prozessor verwendet wird.

5. Eine WC-Anlage nach Anspruch 1 bis 4, wobei als Auffangvorrichtung ein Blas- oder Absaugrohr verwendet wird.
